# EUROPEAN PATENT SPECIFICATION

(11) **EP 1 614 745 B1**
(45) Date of publication and mention of the grant of the patent: **15.07.2009**
(21) Application number: 04723043.8
(22) Date of filing: 24.03.2004
(51) Int. Cl.: C12N 1/21, C12N 9/04, C12N 15/09

(54) **PROCESS FOR PRODUCING GLUCOSE DEHYDROGENASE**
VERFAHREN ZUR HERSTELLUNG VON GLUCOSEDEHYDROGENASE
PROCEDE DE PRODUCTION DE GLUCOSE DESHYDROGENASE

(30) Priority: 25.03.2003 JP 2003082739
(43) Date of publication of application: 11.01.2006
(73) Proprietor: Arkray, Inc., Kyoto-shi, Kyoto 601-8045 (JP)
(72) Inventor: YAMAOKA, Hideaki, Arkray, Inc., Kyoto-shi, Kyoto 6018045 (JP); HOSHIJIMA, Mitsuhiro, Arkray, Inc., Kyoto-shi, Kyoto 6018045 (JP); KAWASE, Shido, Unitika Ltd., Uji-shi, Kyoto 6110021 (JP); KUROSAKA, Keisuke, Unitika Ltd., Uji-shi, Kyoto 6110021 (JP)
(74) Representative: Dean, John Paul
(86) International application number: PCT/JP2004/004074
(87) International publication number: WO 2004/085629

(56) References cited:
- EP-A- 1 498 484
- WO-A1-02/36779
- JP-A- 2003 274 964
- THÖNY-MEIER, L. ET AL: "Escherichia coli genes required for cytochrome c maturation." JOURNAL OF BACTERIOLOGY, vol. 177, no. 15, August 1995 (1995-08), pages 4321-4326, XP002375826
- INOSE K. ET AL: 'Cloning and expression of the gene encoding catalytic subunit of thermostable glucose dehydrogenase from Burkholderia cepacia in Escherichia coli' BIOCHIMICA ET BIOPHYSICA ACTA vol. 1645, no. 2, 21 February 2003, pages 133 - 138, XP004405440
- REINCKE B. ET AL: 'Heterologous expression of soluble fragments of cytochrome c552 acting as electron donor to the Paracoccus denitrificans cytochrome c oxidase' BIOCHIMICA ET BIOPHYSICA ACTA vol. 1411, no. 1, 21 April 1999, pages 114 - 120, XP004338839
- HERBAUD M.-L. ET AL: 'Escherichia coli is able to produce heterologous tetraheme cytochrome c3 when the ccm genes are co-expressed' BIOCHIMICA ET BIOPHYSICA ACTA vol. 1481, no. 1, 31 August 2000, pages 18 - 24, XP004278981

### SEQUENCE LISTING

<110> Arkray, Inc.
<120> Method for producing glucose dehydrogenase
<130> G843-0PC4051
<150> JP 2003-82739
   <151> 2003-03-25
<160> 15
<170> PatentIn Ver. 2.0
<210> 1
   <211> 2467
   <212> DNA
   <213> Burkhorderia cepacia
<220>
   <221> CDS
   <222> (258)..(761)
<220>
   <221> CDS
   <222> (764)..(2380)
<220>
   <221> CDS
   <222> (2386)..(2466)
<400> 1
   aagctttctg tttgattgca cgcgattcta accgagcgtc tgtgaggcgg aacgcgacat 60
   gcttcgtgtc gcacacgtgt cgcgccgacg acacaaaaat gcagcgaaat ggctgatcgt 120
   tacgaatggc tgacacattg aatggactat aaaaccattg tccgttccgg aatgtgcgcg 180
   tacatttcag gtccgcgccg atttttgaga aatatcaagc gtggttttcc cgaatccggt 240
   gttcgagaga aggaaac atg cac aac gac aac act ccc cac tcg cgt cgc    290
                      Met His Asn Asp Asn Thr Pro His Ser Arg Arg
                       1               5                  10
   cac ggc gac gca gcc gca tca ggc atc acg cgg cgt caa tgg ttg caa   338
   His Gly Asp Ala Ala Ala Ser Gly Ile Thr Arg Arg Gln Trp Leu Gln
               15                  20                  25
   ggc gcg ctg gcg ctg acc gca gcg ggc ctc acg ggt tcg ctg aca ttg 386
   Gly Ala Leu Ala Leu Thr Ala Ala Gly Leu Thr Gly Ser Leu Thr Leu
   30 35 40
   cgg gcg ctt gca gac aac ccc ggc act gcg ccg ctc gat acg ttc atg 434
   Arg Ala Leu Ala Asp Asn Pro Gly Thr Ala Pro Leu Asp Thr Phe Met
   45 50 55
   acg ctt tcc gaa tcg ctg acc ggc aag aaa ggg ctc agc cgc gtg atc 482
   Thr Leu Ser Glu Ser Leu Thr Gly Lys Lys Gly Leu Ser Arg Val Ile
   60 65 70 75
   ggc gag cgc ctg ctg cag gcg ctg cag aag ggc tcg ttc aag acg gcc 530
   Gly Glu Arg Leu Leu Gln Ala Leu Gln Lys Gly Ser Phe Lys Thr Ala
   80 85 90
   gac agc ctg ccg cag ctc gcc ggc gcg ctc gcg tcc ggt tcg ctg acg 578
   Asp Ser Leu Pro Gln Leu Ala Gly Ala Leu Ala Ser Gly Ser Leu Thr
   95 100 105
   cct gaa cag gaa tcg ctc gca ctg acg atc ctc gag gcc tgg tat ctc 626
   Pro Glu Gln Glu Ser Leu Ala Leu Thr Ile Leu Glu Ala Trp Tyr Leu
   110 115 120
   ggc atc gtc gac aac gtc gtg att acg tac gag gaa gca tta atg ttc 674
   Gly Ile Val Asp Asn Val Val Ile Thr Tyr Glu Glu Ala Leu Met Phe
   125 130 135
   ggc gtc gtg tcc gat acg ctc gtg atc cgt tcg tat tgc ccc aac aaa 722
   Gly Val Val Ser Asp Thr Leu Val Ile Arg Ser Tyr Cys Pro Asn Lys
   140 145 150 155
   ccc ggc ttc tgg gcc gac aaa ccg atc gag agg caa gcc tg atg gcc 769
   Pro Gly Phe Trp Ala Asp Lys Pro Ile Glu Arg Gln Ala Met Ala
   160 165 170
   gat acc gat acg caa aag gcc gac gtc gtc gtc gtt gga tcg ggt gtc 817
   Asp Thr Asp Thr Gln Lys Ala Asp Val Val Val Val Gly Ser Gly Val
   175 180 185
   gcg ggc gcg atc gtc gcg cat cag ctc gcg atg gcg ggc aag gcg gtg 865
   Ala Gly Ala Ile Val Ala His Gln Leu Ala Met Ala Gly Lys Ala Val
   190 195 200
   atc ctg ctc gaa gcg ggc ccg cgc atg ccg cgc tgg gaa atc gtc gag 913
   Ile Leu Leu Glu Ala Gly Pro Arg Met Pro Arg Trp Glu Ile Val Glu
   205 210 215
   cgc ttc cgc aat cag ccc gac aag atg gac ttc atg gcg ccg tac ccg 961
   Arg Phe Arg Asn Gln Pro Asp Lys Met Asp Phe Met Ala Pro Tyr Pro
   220 225 230
   tcg agc ccc tgg gcg ccg cat ccc gag tac ggc ccg ccg aac gac tac 1009
   Ser Ser Pro Trp Ala Pro His Pro Glu Tyr Gly Pro Pro Asn Asp Tyr
   235 240 245 250
   ctg atc ctg aag ggc gag cac aag ttc aac tcg cag tac atc cgc gcg 1057
   Leu Ile Leu Lys Gly Glu His Lys Phe Asn Ser Gln Tyr Ile Arg Ala
   255 260 265
   gtg ggc ggc acg acg tgg cac tgg gcc gcg tcg gcg tgg cgc ttc att 1105
   Val Gly Gly Thr Thr Trp His Trp Ala Ala Ser Ala Trp Arg Phe Ile
   270 275 280
   ccg aac gac ttc aag atg aag agc gtg tac ggc gtc ggc cgc gac tgg 1153
   Pro Asn Asp Phe Lys Met Lys Ser Val Tyr Gly Val Gly Arg Asp Trp
   285 290 295
   ccg atc cag tac gac gat ctc gag ccg tac tat cag cgc gcg gag gaa 1201
   Pro Ile Gln Tyr Asp Asp Leu Glu Pro Tyr Tyr Gln Arg Ala Glu Glu
   300 305 310
   gag ctc ggc gtg tgg ggc ccg ggc ccc gag gaa gat ctg tac tcg ccg 1249
   Glu Leu Gly Val Trp Gly Pro Gly Pro Glu Glu Asp Leu Tyr Ser Pro
   315 320 325 330
   cgc aag cag ccg tat ccg atg ccg ccg ctg ccg ttg tcg ttc aac gag 1297
   Arg Lys Gln Pro Tyr Pro Met Pro Pro Leu Pro Leu Ser Phe Asn Glu
   335 340 345
   cag acc atc aag acg gcg ctg aac aac tac gat ccg aag ttc cat gtc 1345
   Gln Thr Ile Lys Thr Ala Leu Asn Asn Tyr Asp Pro Lys Phe His Val
   350 355 360
   gtg acc gag ccg gtc gcg cgc aac agc cgc ccg tac gac ggc cgc ccg 1393
   Val Thr Glu Pro Val Ala Arg Asn Ser Arg Pro Tyr Asp Gly Arg Pro
   365 370 375
   act tgt tgc ggc aac aac aac tgc atg ccg atc tgc ccg atc ggc gcg 1441
   Thr Cys Cys Gly Asn Asn Asn Cys Met Pro Ile Cys Pro Ile Gly Ala
   380 385 390
   atg tac aac ggc atc gtg cac gtc gag aag gcc gaa cgc gcc ggc gcg 1489
   Met Tyr Asn Gly Ile Val His Val Glu Lys Ala Glu Arg Ala Gly Ala
   395 400 405 410
   aag ctg atc gag aac gcg gtc gtc tac aag ctc gag acg ggc ccg gac 1537
   Lys Leu Ile Glu Asn Ala Val Val Tyr Lys Leu Glu Thr Gly Pro Asp
   415 420 425
   aag cgc atc gtc gcg gcg ctc tac aag gac aag acg ggc gcc gag cat 1585
   Lys Arg Ile Val Ala Ala Leu Tyr Lys Asp Lys Thr Gly Ala Glu His
   430 435 440
   cgc gtc gaa ggc aag tat ttc gtg ctc gcc gcg aac ggc atc gag acg 1633
   Arg Val Glu Gly Lys Tyr Phe Val Leu Ala Ala Asn Gly Ile Glu Thr
   445 450 455
   ccg aag atc ctg ctg atg tcc gcg aac cgc gat ttc ccg aac ggt gtc 1681
   Pro Lys Ile Leu Leu Met Ser Ala Asn Arg Asp Phe Pro Asn Gly Val
   460 465 470
   gcg aac agc tcg gac atg gtc ggc cgc aac ctg atg gac cat ccg ggc 1729
   Ala Asn Ser Ser Asp Met Val Gly Arg Asn Leu Met Asp His Pro Gly
   475 480 485 490
   acc ggc gtg tcg ttc tat gcg agc gag aag ctg tgg ccg ggc cgc ggc 1777
   Thr Gly Val Ser Phe Tyr Ala Ser Glu Lys Leu Trp Pro Gly Arg Gly
   495 500 505
   ccg cag gag atg acg tcg ctg atc ggt ttc cgc gac ggt ccg ttc cgc 1825
   Pro Gln Glu Met Thr Ser Leu Ile Gly Phe Arg Asp Gly Pro Phe Arg
   510 515 520
   gcg acc gaa gcg gcg aag aag atc cac ctg tcg aac ctg tcg cgc atc 1873
   Ala Thr Glu Ala Ala Lys Lys Ile His Leu Ser Asn Leu Ser Arg Ile
   525 530 535
   gac cag gag acg cag aag atc ttc aag gcc ggc aag ctg atg aag ccc 1921
   Asp Gln Glu Thr Gln Lys Ile Phe Lys Ala Gly Lys Leu Met Lys Pro
   540 545 550
   gac gag ctc gac gcg cag atc cgc gac cgt tcc gca cgc tac gtg cag 1969
   Asp Glu Leu Asp Ala Gln Ile Arg Asp Arg Ser Ala Arg Tyr Val Gln
   555 560 565 570
   ttc gac tgc ttc cac gaa atc ctg ccg caa ccc gag aac cgc atc gtg 2017
   Phe Asp Cys Phe His Glu Ile Leu Pro Gln Pro Glu Asn Arg Ile Val
   575 580 585
   ccg agc aag acg gcg acc gat gcg atc ggc att ccg cgc ccc gag atc 2065
   Pro Ser Lys Thr Ala Thr Asp Ala Ile Gly Ile Pro Arg Pro Glu Ile
   590 595 600
   acg tat gcg atc gac gac tac gtg aag cgc ggc gcc gcg cat acg cgc 2113
   Thr Tyr Ala Ile Asp Asp Tyr Val Lys Arg Gly Ala Ala His Thr Arg
   605 610 615
   gag gtc tac gcg acc gcc gcg aag gtg ctc ggc ggc acg gac gtc gtg 2161
   Glu Val Tyr Ala Thr Ala Ala Lys Val Leu Gly Gly Thr Asp Val Val
   620 625 630
   ttc aac gac gaa ttc gcg ccg aac aat cac atc acg ggc tcg acg atc 2209
   Phe Asn Asp Glu Phe Ala Pro Asn Asn His Ile Thr Gly Ser Thr Ile
   635 640 645 650
   atg ggc gcc gat gcg cgc gac tcc gtc gtc gac aag gac tgc cgc acg 2257
   Met Gly Ala Asp Ala Arg Asp Ser Val Val Asp Lys Asp Cys Arg Thr
   655 660 665
   ttc gac cat ccg aac ctg ttc att tcg agc agc gcg acg atg ccg acc 2305
   Phe Asp His Pro Asn Leu Phe Ile Ser Ser Ser Ala Thr Met Pro Thr
   670 675 680
   gtc ggt acc gta aac gtg acg ctg acg atc gcc gcg ctc gcg ctg cgg 2353
   Val Gly Thr Val Asn Val Thr Leu Thr Ile Ala Ala Leu Ala Leu Arg
   685 690 695
   atg tcg gac acg ctg aag aag gaa gtc tgacc gtg cgg aaa tct act ctc 2403
   Met Ser Asp Thr Leu Lys Lys Glu Val Val Arg Lys Ser Thr Leu
   700 705 710
   act ttc ctc atc gcc ggc tgc ctc gcg ttg ccg ggc ttc gcg cgc gcg 2451
   Thr Phe Leu Ile Ala Gly Cys Leu Ala Leu Pro Gly Phe Ala Arg Ala
   715 720 725
   gcc gat gcg gcc gat c 2467
   Ala Asp Ala Ala Asp
   730
<210> 2
   <211> 168
   <212> PRT
   <213> Burkhorderia cepacia
<400> 2
   Met His Asn Asp Asn Thr Pro His Ser Arg Arg His Gly Asp Ala Ala
   1 5 10 15
   Ala Ser Gly Ile Thr Arg Arg Gln Trp Leu Gln Gly Ala Leu Ala Leu
   20 25 30
   Thr Ala Ala Gly Leu Thr Gly Ser Leu Thr Leu Arg Ala Leu Ala Asp
   35 40 45
   Asn Pro Gly Thr Ala Pro Leu Asp Thr Phe Met Thr Leu Ser Glu Ser
   50 55 60
   Leu Thr Gly Lys Lys Gly Leu Ser Arg Val Ile Gly Glu Arg Leu Leu
   65 70 75 80
   Gln Ala Leu Gln Lys Gly Ser Phe Lys Thr Ala Asp Ser Leu Pro Gln
   85 90 95
   Leu Ala Gly Ala Leu Ala Ser Gly Ser Leu Thr Pro Glu Gln Glu Ser
   100 105 110
   Leu Ala Leu Thr Ile Leu Glu Ala Trp Tyr Leu Gly Ile Val Asp Asn
   115 120 125
   Val Val Ile Thr Tyr Glu Glu Ala Leu Met Phe Gly Val Val Ser Asp
   130 135 140
   Thr Leu Val Ile Arg Ser Tyr Cys Pro Asn Lys Pro Gly Phe Trp Ala
   145 150 155 160
   Asp Lys Pro Ile Glu Arg Gln Ala
   165
<210> 3
   <211> 539
   <212> PRT
   <213> Burkhorderia cepacia
<400> 3
   Met Ala Asp Thr Asp Thr Gln Lys Ala Asp Val Val Val Val Gly Ser
   1 5 10 15
   Gly Val Ala Gly Ala Ile Val Ala His Gln Leu Ala Met Ala Gly Lys
   20 25 30
   Ala Val Ile Leu Leu Glu Ala Gly Pro Arg Met Pro Arg Trp Glu Ile
   35 40 45
   Val Glu Arg Phe Arg Asn Gln Pro Asp Lys Met Asp Phe Met Ala Pro
   50 55 60
   Tyr Pro Ser Ser Pro Trp Ala Pro His Pro Glu Tyr Gly Pro Pro Asn
   65 70 75 80
   Asp Tyr Leu Ile Leu Lys Gly Glu His Lys Phe Asn Ser Gln Tyr Ile
   85 90 95
   Arg Ala Val Gly Gly Thr Thr Trp His Trp Ala Ala Ser Ala Trp Arg
   100 105 110
   Phe Ile Pro Asn Asp Phe Lys Met Lys Ser Val Tyr Gly Val Gly Arg
   115 120 125
   Asp Trp Pro Ile Gln Tyr Asp Asp Leu Glu Pro Tyr Tyr Gln Arg Ala
   130 135 140
   Glu Glu Glu Leu Gly Val Trp Gly Pro Gly Pro Glu Glu Asp Leu Tyr
   145 150 155 160
   Ser Pro Arg Lys Gln Pro Tyr Pro Met Pro Pro Leu Pro Leu Ser Phe
   165 170 175
   Asn Glu Gln Thr Ile Lys Thr Ala Leu Asn Asn Tyr Asp Pro Lys Phe
   180 185 190
   His Val Val Thr Glu Pro Val Ala Arg Asn Ser Arg Pro Tyr Asp Gly
   195 200 205
   Arg Pro Thr Cys Cys Gly Asn Asn Asn Cys Met Pro Ile Cys Pro Ile
   210 215 220
   Gly Ala Met Tyr Asn Gly Ile Val His Val Glu Lys Ala Glu Arg Ala
   225 230 235 240
   Gly Ala Lys Leu Ile Glu Asn Ala Val Val Tyr Lys Leu Glu Thr Gly
   245 250 255
   Pro Asp Lys Arg Ile Val Ala Ala Leu Tyr Lys Asp Lys Thr Gly Ala
   260 265 270
   Glu His Arg Val Glu Gly Lys Tyr Phe Val Leu Ala Ala Asn Gly Ile
   275 280 285
   Glu Thr Pro Lys Ile Leu Leu Met Ser Ala Asn Arg Asp Phe Pro Asn
   290 295 300
   Gly Val Ala Asn Ser Ser Asp Met Val Gly Arg Asn Leu Met Asp His
   305 310 315 320
   Pro Gly Thr Gly Val Ser Phe Tyr Ala Ser Glu Lys Leu Trp Pro Gly
   325 330 335
   Arg Gly Pro Gln Glu Met Thr Ser Leu Ile Gly Phe Arg Asp Gly Pro
   340 345 350
   Phe Arg Ala Thr Glu Ala Ala Lys Lys Ile His Leu Ser Asn Leu Ser
   355 360 365
   Arg Ile Asp Gln Glu Thr Gln Lys Ile Phe Lys Ala Gly Lys Leu Met
   370 375 380
   Lys Pro Asp Glu Leu Asp Ala Gln Ile Arg Asp Arg Ser Ala Arg Tyr
   385 390 395 400
   Val Gln Phe Asp Cys Phe His Glu Ile Leu Pro Gln Pro Glu Asn Arg
   405 410 415
   Ile Val Pro Ser Lys Thr Ala Thr Asp Ala Ile Gly Ile Pro Arg Pro
   420 425 430
   Glu Ile Thr Tyr Ala Ile Asp Asp Tyr Val Lys Arg Gly Ala Ala His
   435 440 445
   Thr Arg Glu Val Tyr Ala Thr Ala Ala Lys Val Leu Gly Gly Thr Asp
   450 455 460
   Val Val Phe Asn Asp Glu Phe Ala Pro Asn Asn His Ile Thr Gly Ser
   465 470 475 480
   Thr Ile Met Gly Ala Asp Ala Arg Asp Ser Val Val Asp Lys Asp Cys
   485 490 495
   Arg Thr Phe Asp His Pro Asn Leu Phe Ile Ser Ser Ser Ala Thr Met
   500 505 510
   Pro Thr Val Gly Thr Val Asn Val Thr Leu Thr Ile Ala Ala Leu Ala
   515 520 525
   Leu Arg Met Ser Asp Thr Leu Lys Lys Glu Val
   530 535
<210> 4
   <211> 27
   <212> PRT
   <213> Burkhorderia cepacia
<400> 4
   Val Arg Lys Ser Thr Leu Thr Phe Leu Ile Ala Gly Cys Leu Ala Leu
   1 5 10 15
   Pro Gly Phe Ala Arg Ala Ala Asp Ala Ala Asp
   20 25
<210> 5
   <210> 16
   <212> PRT
   <213> Burkhorderia cepacia
<400> 5
<210> 6
   <211> 25
   <212> PRT
   <213> Artificial Sequence
<220>
   <223> Description of Artificial Sequence:consensus
<220>
   <221> UNSURE
   <222> (6, 17, 18, 19, 22)
   <223> Xaa=unknown
<400> 6
   Ala Asp Ala Ala Asp Xaa Ala Leu Val Lys Arg Gly Glu Tyr Leu Ala
   1 5 10 15
   Xaa Xaa Xaa Asp Cys Xaa Ala Cys His
   20 25
<210> 7
   <211> 27
   <212> DNA
   <213> Artificial Sequence
<220>
   <223> Description of Artificial Sequence: primer
<400> 7
   tgcaccgtgc ggaaatctac tctcact 27
<210> 8
   <211> 27
   <212> DNA
   <213> Artificial Sequence
<220>
   <223> Description of Artificial Sequence: primer
<400> 8
   acttccttct tcagcgtgtc cgacatc 27
<210> 9
   <211> 1441
   <212> DNA
   <213> Burkholderia cepacia
<220>
   <221> CDS
   <222> (121)..(1398)
<400> 9
   tccgaacctg ttcatttcga gcagcgcgac gatgccgacc gtcggtaccg taaacgtgac 60
   gctgacgatc gccgcgctcg cgctgcggat gtcggacacg ctgaagaagg aagtctgacc 120
   gtg cgg aaa tct act ctc act ttc ctc atc gcc ggc tgc ctc gcg ttg   168
   Val Arg Lys Ser Thr Leu Thr Phe Leu Ile Ala Gly Cys Leu Ala Leu
    1               5                  10                  15
   ccg ggc ttc gcg cgc gcg gcc gat gcg gcc gat ccg gcg ctg gtc aag   216
   Pro Gly Phe Ala Arg Ala Ala Asp Ala Ala Asp Pro Ala Leu Val Lys
               20                  25                  30
   cgc ggc gaa tac ctc gcg acc gcc atg ccg gta ccg atg ctc ggc aag   264
   Arg Gly Glu Tyr Leu Ala Thr Ala Met Pro Val Pro Met Leu Gly Lys
           35                  40                  45
   atc tac acg agc aac atc acg ccc gat ccc gat acg ggc gac tgc atg   312
   Ile Tyr Thr Ser Asn Ile Thr Pro Asp Pro Asp Thr Gly Asp Cys Met
       50                  55                  60
   gcc tgc cac acc gtg aag ggc ggc aag ccg tac gcg ggc ggc ctt ggc   360
   Ala Cys His Thr Val Lys Gly Gly Lys Pro Tyr Ala Gly Gly Leu Gly
   65                  70                  75                  80
   ggc atc ggc aaa tgg acg ttc gag gac ttc gag cgc gcg gtg cgg cac   408
   Gly Ile Gly Lys Trp Thr Phe Glu Asp Phe Glu Arg Ala Val Arg His
                   85                  90                  95
   ggc gtg tcg aag aac ggc gac aac ctg tat ccg gcg atg ccg tac gtg   456
   Gly Val Ser Lys Asn Gly Asp Asn Leu Tyr Pro Ala Met Pro Tyr Val
               100                 105                 110
   tcg tac gcg aag atc aag gac gac gac gta cgc gcg ctg tac gcc tac   504
   Ser Tyr Ala Lys Ile Lys Asp Asp Asp Val Arg Ala Leu Tyr Ala Tyr
           115                 120                 125
   ttc atg cac ggc gtc gag ccg gtc aag cag gcg ccg ccg aag aac gag   552
   Phe Met His Gly Val Glu Pro Val Lys Gln Ala Pro Pro Lys Asn Glu
       130                 135                 140
   atc cca gcg ctg cta agc atg cgc tgg ccg ctg aag atc tgg aac tgg   600
   Ile Pro Ala Leu Leu Ser Met Arg Trp Pro Leu Lys Ile Trp Asn Trp
   145                 150                 155                 160
   ctg ttc ctg aag gac ggc ccg tac cag ccg aag ccg tcg cag agc gcc   648
   Leu Phe Leu Lys Asp Gly Pro Tyr Gln Pro Lys Pro Ser Gln Ser Ala
                       165                     170                     175
   gaa tgg aat cgc ggc gcg tat ctg gtg cag ggt ctc gcg cac tgc agc      696
   Glu Trp Asn Arg Gly Ala Tyr Leu Val Gln Gly Leu Ala His Cys Ser
                   180                     185                     190
   acg tgc cac acg ccg cgc ggc atc gcg atg cag gag aag tcg ctc gac      744
   Thr Cys His Thr Pro Arg Gly Ile Ala Met Gln Glu Lys Ser Leu Asp
               195                     200                     205
   gaa acc ggc ggc agc ttc ctc gcg ggg tcg gtg ctc gcc ggc tgg gac      792
   Glu Thr Gly Gly Ser Phe Leu Ala Gly Ser Val Leu Ala Gly Trp Asp
           210                     215                     220
   ggc tac aac atc acg tcg gac ccg aat gcg ggg atc ggc agc tgg acg      840
   Gly Tyr Asn Ile Thr Ser Asp Pro Asn Ala Gly Ile Gly Ser Trp Thr
   225                     230                     235                     240
   cag cag cag ctc gtg cag tat ttg cgc acc ggc agc gtg ccg ggc gtc      888
   Gln Gln Gln Leu Val Gln Tyr Leu Arg Thr Gly Ser Val Pro Gly Val
                       245                     250                     255
   gcg cag gcg gcc ggg ccg atg gcc gag gcg gtc gag cac agc ttc tcg      936
   Ala Gln Ala Ala Gly Pro Met Ala Glu Ala Val Glu His Ser Phe Ser
                   260                     265                     270
   aag atg acc gaa gcg gac atc ggt gcg atc gcc acg tac gtc cgc acg      984
   Lys Met Thr Glu Ala Asp Ile Gly Ala Ile Ala Thr Tyr Val Arg Thr
               275                     280                     285
   gtg ccg gcc gtt gcc gac agc aac gcg aag cag ccg cgg tcg tcg tgg     1032
   Val Pro Ala Val Ala Asp Ser Asn Ala Lys Gln Pro Arg Ser Ser Trp
           290                     295                     300
   ggc aag ccg gcc gag gac ggg ctg aag ctg cgc ggt gtc gcg ctc gcg     1080
   Gly Lys Pro Ala Glu Asp Gly Leu Lys Leu Arg Gly Val Ala Leu Ala
   305                     310                     315                     320
   tcg tcg ggc atc gat ccg gcg cgg ctg tat ctc ggc aac tgc gcg acg     1128
   Ser Ser Gly Ile Asp Pro Ala Arg Leu Tyr Leu Gly Asn Cys Ala Thr
                       325                     330                     335
   tgc cac cag atg cag ggc aag ggc acg ccg gac ggc tat tac ccg tcg     1176
   Cys His Gln Met Gln Gly Lys Gly Thr Pro Asp Gly Tyr Tyr Pro Ser
                   340                     345                     350
   ctg ttc cac aac tcc acc gtc ggc gcg tcg aat ccg tcg aac ctc gtg     1224
   Leu Phe His Asn Ser Thr Val Gly Ala Ser Asn Pro Ser Asn Leu Val
               355                     360                     365
   cag gtg atc ctg aac ggc gtg cag cgc aag atc ggc agc gag gat atc     1272
   Gln Val Ile Leu Asn Gly Val Gln Arg Lys Ile Gly Ser Glu Asp Ile
           370                     375                     380
   ggg atg ccc gct ttc cgc tac gat ctg aac gac gcg cag atc gcc gcg     1320
   Gly Met Pro Ala Phe Arg Tyr Asp Leu Asn Asp Ala Gln Ile Ala Ala
   385                     390                     395                     400
   ctg acg aac tac gtg acc gcg cag ttc ggc aat ccg gcg gcg aag gtg 1368
   Leu Thr Asn Tyr Val Thr Ala Gln Phe Gly Asn Pro Ala Ala Lys Val
   405 410 415
   acg gag cag gac gtc gcg aag ctg cgc tga catagtcggg cgcgccgaca 1418
   Thr Glu Gln Asp Val Ala Lys Leu Arg
   420 425
   cggcgcaacc gataggacag gag 1441
<210> 10
   <211> 425
   <212> PRT
   <213> Burkholderia cepacia
<400> 10
   Val Arg Lys Ser Thr Leu Thr Phe Leu Ile Ala Gly Cys Leu Ala Leu
   1 5 10 15
   Pro Gly Phe Ala Arg Ala Ala Asp Ala Ala Asp Pro Ala Leu Val Lys
   20 25 30
   Arg Gly Glu Tyr Leu Ala Thr Ala Met Pro Val Pro Met Leu Gly Lys
   35 40 45
   Ile Tyr Thr Ser Asn Ile Thr Pro Asp Pro Asp Thr Gly Asp Cys Met
   50 55 60
   Ala Cys His Thr Val Lys Gly Gly Lys Pro Tyr Ala Gly Gly Leu Gly
   65 70 75 80
   Gly Ile Gly Lys Trp Thr Phe Glu Asp Phe Glu Arg Ala Val Arg His
   85 90 95
   Gly Val Ser Lys Asn Gly Asp Asn Leu Tyr Pro Ala Met Pro Tyr Val
   100 105 110
   Ser Tyr Ala Lys Ile Lys Asp Asp Asp Val Arg Ala Leu Tyr Ala Tyr
   115 120 125
   Phe Met His Gly Val Glu Pro Val Lys Gln Ala Pro Pro Lys Asn Glu
   130 135 140
   Ile Pro Ala Leu Leu Ser Met Arg Trp Pro Leu Lys Ile Trp Asn Trp
   145 150 155 160
   Leu Phe Leu Lys Asp Gly Pro Tyr Gln Pro Lys Pro Ser Gln Ser Ala
   165 170 175
   Glu Trp Asn Arg Gly Ala Tyr Leu Val Gln Gly Leu Ala His Cys Ser
   180 185 190
   Thr Cys His Thr Pro Arg Gly Ile Ala Met Gln Glu Lys Ser Leu Asp
   195 200 205
   Glu Thr Gly Gly Ser Phe Leu Ala Gly Ser Val Leu Ala Gly Trp Asp
   210 215 220
   Gly Tyr Asn Ile Thr Ser Asp Pro Asn Ala Gly Ile Gly Ser Trp Thr
   225 230 235 240
   Gln Gln Gln Leu Val Gln Tyr Leu Arg Thr Gly Ser Val Pro Gly Val
   245 250 255
   Ala Gln Ala Ala Gly Pro Met Ala Glu Ala Val Glu His Ser Phe Ser
   260 265 270
   Lys Met Thr Glu Ala Asp Ile Gly Ala Ile Ala Thr Tyr Val Arg Thr
   275 280 285
   Val Pro Ala Val Ala Asp Ser Asn Ala Lys Gln Pro Arg Ser Ser Trp
   290 295 300
   Gly Lys Pro Ala Glu Asp Gly Leu Lys Leu Arg Gly Val Ala Leu Ala
   305 310 315 320
   Ser Ser Gly Ile Asp Pro Ala Arg Leu Tyr Leu Gly Asn Cys Ala Thr
   325 330 335
   Cys His Gln Met Gln Gly Lys Gly Thr Pro Asp Gly Tyr Tyr Pro Ser
   340 345 350
   Leu Phe His Asn Ser Thr Val Gly Ala Ser Asn Pro Ser Asn Leu Val
   355 360 365
   Gln Val Ile Leu Asn Gly Val Gln Arg Lys Ile Gly Ser Glu Asp Ile
   370 375 380
   Gly Met Pro Ala Phe Arg Tyr Asp Leu Asn Asp Ala Gln Ile Ala Ala
   385 390 395 400
   Leu Thr Asn Tyr Val Thr Ala Gln Phe Gly Asn Pro Ala Ala Lys Val
   405 410 415
   Thr Glu Gln Asp Val Ala Lys Leu Arg
   420 425
<210> 11
   <211> 5
   <212> PRT
   <213> Artificial Sequence
<220>
   <223> Description of Artificial Sequence: heme binding motif
<220>
   <221> UNSURE
   <222> (2,3)
   <223> Xaa=unknown
<400> 11
   Cys Xaa Xaa Cys His
   1 5
<210> 12
   <211> 26
   <212> DNA
   <213> Artificial Sequence
<220>
   <223> Description of Artificial Sequence: primer
<400> 12
   catgccatgg cacacaacga caacac 26
<210> 13
   <211> 30
   <212> DNA
   <213> Artificial Sequence
<220>
   <223> Description of Artificial Sequence: primer
<400> 13
   gtcgacgatc ttcttccagc cgaacatcac 30
<210> 14
   <211> 30
   <212> DNA
   <213> Artificial Sequence
<220>
   <223> Description of Artificial Sequence: primer
<400> 14
   tggccatggt tgaagccaga gagttacttt 30
<210> 15
   <211> 30
   <212> DNA
   <213> Artificial Sequence
<220>
   <223> Description of Artificial Sequence: primer
<400> 15
   ttatttactc tcctgcggcg acaaatgttg 30

## Description

### Technical Field

The present invention relates to a method for producing an *Escherichia* bacterium that abundantly expresses a glucose dehydrogenase complex of *Burkholderia cepacia* and a method for producing the enzyme complex. Glucose dehydrogenase is useful in a glucose sensor using an enzyme electrode and so forth.

### Background Art

As a glucose dehydrogenase (GDH), an enzyme produced by a microorganism belonging to the genus *Burkholderia* (*Burkholderia cepacia* KS1 strain) has been known. This enzyme is a complex consisting of the α-subunit, which is a catalyst subunit, the β-subunit, which is cytochrome c, and the γ-subunit, and has superior properties that it has high thermal stability, the reaction catalyzed by it is hardly affected by dissolved oxygen, and so forth. DNAs encoding the α-subunit and the γ-subunit of this enzyme have been isolated, and a part of a DNA encoding the β-subunit has also been isolated. Furthermore, this enzyme has been successfully expressed in *Escherichia coli* (refer to International Patent Publication WO02/36779; EP-A 1331272). However, the expressed GDH does not appear to contain the β-subunit. EP-A 1498484 discloses an *E. coli* bacterium into which has been introduced DNA encoding the α subunit or the β subunit of glucose dehydrogenase of *B. cepacia* in an expressible form.

Meanwhile, as a cytochrome maturation system of *Escherichia coli,* the ccm system (cytochrome c maturation system) is known. The ccm system is known to be expressed in *Escherichia coli* under anaerobic and special conditions (J. Bacteriol. 177, 4321-4326 (1995)). The DNA sequence of the operon encoding the ccm system (*ccmABCDEFGH*) has already been elucidated (Nature, 409 (6819), 529-533 (2001); GeneBank database accession U0008 (1993)). Furthermore, it has been reported that a covalently bonded type multi-heme cytochrome derived from an organism of a species different from *Escherichia coli* was expressed and produced under an aerobic condition in *Escherichia coli* transformed with a plasmid that expresses the ccm system (Biochem. Biophys. Res. Commun., 251, 744-7 (1998); Biochem. Biophys. Acta, 1411, 114-20 (1999); Biochem. Biophys. Acta, 1481, 18-24 (2000); Protein Sci. 9, 2074-84 (2000)).

Furthermore, as a plasmid containing the *ccm* operon, the plasmid pEC86 obtained by inserting the operon into pACYC184 is known (Biochem. Biophys. Res. Commun., 251, 744-7 (1998)).

### Disclosure of the Invention

The inventors of the present invention previously found that the expression efficiency was lower when the α-subunit and the β-subunit of the aforementioned glucose dehydrogenase were simultaneously expressed in *Escherichia coli* than when the α-subunit alone was expressed. Thus, an object of the present invention is to provide a means for abundantly expressing an enzyme complex containing the α-subunit and the β-subunit in an *Escherichia* bacterium.

The inventors of the present invention assiduously studied in order to achieve the foregoing object. As a result, they found that expression of a DNA encoding a glucose dehydrogenase complex of *Burkholderia cepacia* could be improved in an *Escherichia* bacterium by enhancing the expression of the ccm system of the bacterium, and thus accomplished the present invention.

The present invention thus provides the followings.
(1) An *Escherichia* bacterium, into which is introduced DNA encoding the α-subunit and the β-subunit of glucose dehydrogenase (GDH) of *Burkholderia cepacia* in an expressible form and a plasmid containing the cytochrome c maturation (ccm) operon of *Escherichia coli* ligated to a suitable promoter which constitutively expresses the operon, wherein expression of the ccm system is enhanced, as compared with a wild-type strain or unmodified strain of *Escherichia* bacteria.
(2) The *Escherichia* bacterium according to (1), wherein the DNA encoding the α-subunit locates upstream from the DNA encoding the β-subunit, and expressions of them are regulated by a single promoter.
(3) The *Escherichia* bacterium according to (1), which is further introduced with a DNA encoding the γ-subunit of the glucose dehydrogenase in an expressible form.
(4) The *Escherichia* bacterium according to (3), wherein the DNA encoding the γ-subunit locates upstream from the DNA encoding the α-subunit.
(5) The *Escherichia* bacterium according to any one of (1) to (4), wherein the *Escherichia* bacterium is *Escherichia coli.*
(6) A method for producing a glucose dehydrogenase complex, which comprises culturing the *Escherichia* bacterium according to any one of (1) to (5) so that the DNAs encoding the α-subunit and the β-subunit are expressed and the glucose dehydrogenase complex is produced and, collecting the complex.

### Brief Description of the Drawings

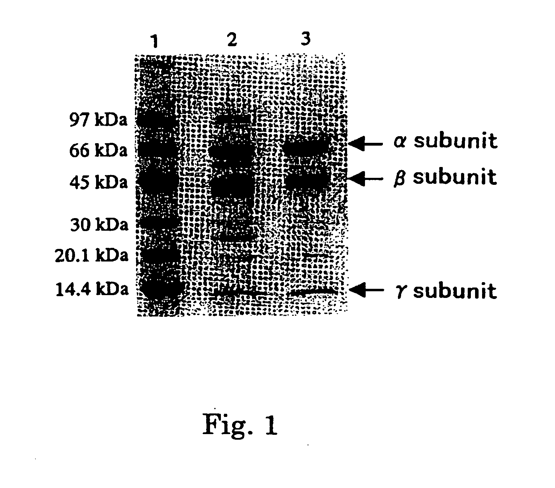

Fig. 1 shows results of SDS-PAGE of GDH complexes purified from the *Burkholderia cepacia* KS1 strain and the *Escherichia coli* JM109/pTrc99Aγαβ,pBBJMccm (photograph).
Lane 1: Marker
Lane 2: GDH purified from the KS1 strain
Lane 3: GDH purified from JM109/pTrc99Aγαβ, pBBJMccm

### Best Mode for Carrying out the Invention

Hereafter, the present invention will be explained in detail.

The *Escherichia* bacterium of the present invention is an *Escherichia* bacterium into which DNAs encoding the α-subunit and the β-subunit (hereinafter, also referred to as "α-subunit gene" and "β-subunit gene", respectively) of glucose dehydrogenase (hereinafter, also referred to simply as "GDH") of *Burkholderia cepacia* are introduced in an expressible form, and in which the ccm system is enhanced.

Examples of the aforementioned *Escherichia* bacterium include *Escherichia coli.*

Furthermore, examples of the aforementioned *Burkholderia cepacia* include the KS1 strain, JCM2800 strain, JCM2801 strain, J2315 strain and so forth. The KS1 strain was deposited at the International Patent Organism Depositary, National Institute of Advanced Industrial Science and Technology (Tsukuba Central 6, 1-1, Higashi 1-Chome, Tsukuba-shi, Ibaraki-ken, 305-8566, Japan) and given an accession number FERM BP-7306. The JCM2800 strain and the JCM2801 strain are available from the Riken Bioresource Center, Japan Collection of Microorganisms (JCM). Further, the J2315 strain was deposited at the American Type Culture Collection (ATCC) with an ATCC number BAA-245 and the Belgian Co-ordinated Collections of Micro-organisms (BCCMTM) with an accession number LNG 16656, and is available from these depositories.

A nucleotide sequence of a chromosomal DNA fragment containing the α-subunit gene and a part of the β-subunit gene of GDH of the KS1 strain is shown in SEQ ID NO: 1 (refer to WO02/36779). Three open reading frames (ORFs) exist in this nucleotide sequence. The second and third ORFs from the 5' end encode the α-subunit (SEQ ID NO: 3) and the β-subunit (SEQ ID NO: 4), respectively. Furthermore, the first ORF is estimated to encode the γ-subunit (SEQ ID NO: 2). A nucleotide sequence of a fragment containing the full-length β-subunit gene is shown in SEQ ID NO: 9. Furthermore, the amino acid sequence of the β-subunit is shown in SEQ ID NO: 10. In SEQ ID NO: 10, the amino acid numbers 1 to 22 are estimated to be a signal peptide. Although the first amino acid residue is indicated as Val in SEQ ID NOS: 9 and 10, it is very likely to be Met, and may be removed after translation.

The α-subunit gene used in the present invention is not limited to a gene encoding the amino acid sequence of SEQ ID NO: 3, and may be one encoding a protein having an amino acid sequence including substitution, deletion, insertion or addition of one or several amino acid residues in the amino acid sequence of SEQ ID NO: 3 so long as the encoded polypeptide has the GDH activity. Although an amino acid sequence that can be encoded by the nucleotide sequence of SEQ ID NO: 1 is shown in SEQ ID NO: 3, the methionine residue at the N-terminus may be removed after translation. The expression "one or several" mentioned above means preferably a number of 1 to 10, more preferably 1 to 5, particularly preferably 1 to 3.

Furthermore, the β-subunit gene may encode a protein having an amino acid sequence including substitution, deletion, insertion or addition of one or several amino acid residues in the amino acid sequence of the amino acid numbers 23 to 425 of SEQ ID NO: 10 so long as it functions as the β-subunit of GDH. The expression "one or several" mentioned above means preferably a number of 1 to 20, more preferably 1 to 10, particularly preferably 1 to 5. The expression "function as the β-subunit of GDH" means to function as cytochrome c without impairing the enzymatic activity of GDH.

Specific examples of the α-subunit gene include a DNA comprising the nucleotide sequence of the nucleotide numbers 764 to 2380 of SEQ ID NO: 1. Furthermore, the α-subunit gene may be a DNA hybridizable with a DNA comprising the nucleotide sequence of the nucleotide numbers 764 to 2380 of SEQ ID NO: 1 or a probe prepared from this sequence under stringent conditions and encoding a protein having the GDH activity.

Furthermore, specific examples of the β-subunit gene include a DNA comprising the nucleotide sequence of the nucleotide numbers 187 to 1398 of SEQ ID NO: 9. Furthermore, the β-subunit gene may be a DNA hybridizable with a DNA comprising the nucleotide sequence of the nucleotide numbers 187 to 1398 of SEQ ID NO: 9 or a probe prepared from this sequence under a stringent condition and encoding a protein that can function as the β-subunit.

The aforementioned stringent conditions include a condition under which DNAs having a homology of 70% or more, preferably 80% or more, more preferably 90% or more, hybridize with each other. Specifically, a condition of 1 x SSC and 0.1% SDS at 60°C can be mentioned.

The α-subunit gene and the β-subunit gene can be obtained by, for example, PCR using a chromosomal DNA of the *Burkholderia cepacia* KS1 strain as a template. Primers for PCR can be prepared by chemical synthesis on the basis of the aforementioned nucleotide sequences. Furthermore, they can also be obtained from chromosomal DNA of the *Burkholderia cepacia* KS1 strain by hybridization using an oligonucleotide prepared on the basis of the aforementioned sequences as a probe. Furthermore, their variants can also be obtained in the same manner from other *Burkholderia cepacia* strains.

In the present invention, it is preferred that the α-subunit gene locates upstream from the β-subunit gene, and that their expressions are both regulated by a single promoter. Furthermore, it is preferred that a DNA encoding the γ-subunit (γ-subunit gene) is introduced into an *Escherichia* bacterium in an expressible form together with the α-subunit gene and the β-subunit gene. In this case, it is preferred that the γ-subunit gene locates upstream from the α-subunit gene, and that expressions of the subunit genes are all regulated by a single promoter.

A polycistronic DNA fragment encoding the γ-subunit, α-subunit and β-subunit in this order can be obtained by, for example, PCR using a chromosomal DNA of the *Burkholderia cepacia* KS1 strain as a template and oligonucleotides having the nucleotide sequences of SEQ ID NOS: 12 and 13 as primers (see the examples described later).

To introduce a DNA containing the α-subunit gene and the β-subunit gene (and the γ-subunit gene as required) (hereinafter, referred to as "GDHαβ gene") into an *Escherichia* bacterium in an expressible form, the GDHαβ gene can be inserted into a vector that functions in the *Escherichia* bacterium, and the *Escherichia* bacterium can be transformed with the obtained recombinant vector. In this case, by providing a promoter functioning in the *Escherichia* bacterium upstream from the GDHαβ gene, the GDHαβ gene can be expressed under the regulation by this promoter.

Examples of the vector that functions in an *Escherichia* bacterium include pBR322, pUC18, pUC118, pUC19, pUC119, pACYC184, pBBR122 and so forth. Furthermore, examples of the aforementioned promoter include lac, trp, tac, trc, P_{L}, tet, PhoA and so forth. Furthermore, by inserting the GDHαβ gene into an expression vector containing a promoter at a suitable site, the insertion of the gene into the vector and the ligation of the promoter can be performed in the same step. Examples of such an expression vector include pTrc99A, pBluescript, pKK223-3 and so forth.

Furthermore, the GDHαβ gene may be incorporated into a chromosomal DNA of an *Escherichia* bacterium in an expressible form.

To transform an *Escherichia* bacterium with a recombinant vector, the competent cell method using a calcium treatment, electroporation or the like can be employed, for example.

The *Escherichia* bacterium of the present invention is an *Escherichia* bacterium which is introduced with the GDHαβ gene as described above, and in which expression of the ccm system is enhanced.

The ccm system is encoded by the *ccm* operon (*ccmABCDEFGH*). The *ccm* operon can be obtained by, for example, PCR using a chromosomal DNA of *Escherichia coli* as a template. Primers for PCR can be prepared by synthesis on the basis of the reported nucleotide sequence (DDBJ/EMBL/GenBank accession number AE005452). Furthermore, they can be obtained from a chromosomal DNA of *Escherichia coli* by hybridization using an oligonucleotide prepared on the basis of the aforementioned sequence as a probe. Furthermore, their variants can also be obtained in the same manner from other *Escherichia* bacteria.

The *ccm* operon may be, in addition to the DNA having the aforementioned reported nucleotide sequence, a DNA hybridizable with the DNA comprising the nucleotide sequence or a probe prepared from the sequence under stringent conditions and encoding a group of enzymes functioning as the ccm system.

The ccm system is known to be expressed under anaerobic and special conditions (non-patent document 1). In the present invention, the expression "expression of the ccm system is enhanced" means that the expression is enhanced compared with that in a wild strain or unmodified strain of *Escherichia* bacteria, or that a modification is made so that the system can be expressed even under conditions which are not the anaerobic and special conditions. Examples of conditions which are not the anaerobic and special conditions include an aerobic condition.

To enhance the expression of the ccm system, genes of the *ccm* operon can be ligated to a promoter that constitutively expresses them or a promoter that can regulate their expressions, and the obtained recombinant gene can be introduced into an *Escherichia* bacterium. Preferred promoters and vectors, and introduction of the *ccm* operon into an *Escherichia* bacterium are similar to those described with regard to the GDHαβ gene.

Examples of a plasmid containing the *ccm operon* include pEC86 obtained by inserting the operon into pACYC184. This operon is constitutively expressed under regulation by the tet promoter. Furthermore, a plasmid into which the *ccm* operon cloned by PCR or the like from a chromosomal DNA of *Escherichia* bacterium is inserted so as to be under control of a suitable promoter can also be prepared.

Furthermore, the expression of the operon can also be enhanced by replacing the promoter of the *ccm* operon on a chromosomal DNA of *Escherichia* bacterium with a suitable promoter.

The GDH complex can be efficiently produced by culturing the *Escherichia* bacterium of the present invention to allow the bacterium to express the α-subunit gene and the β-subunit gene and produce the GDH enzyme complex as an expression product of these and collecting the complex. The term "GDH complex" used here preferably means a multimeric protein formed by association of the subunits, but also includes a mixture of the subunits in free forms.

As for the method of culturing the *Escherichia* bacterium, culture conditions can be selected taking nutritional and physiological properties of a host into consideration. The culture is performed as liquid culture in many cases. Industrial culture is advantageously performed with aeration by stirring.

As the nutrients of the medium, those usually used for culture of *Escherichia* bacteria can be extensively used. As the carbon source, a carbon compound that can be assimilated may be used, and for example, glucose, sucrose, lactose, maltose, galactose, molasses, pyruvic acid and so forth are used. As the nitrogen source, a nitrogen compound that may be utilized, and for example, peptone, meat extract, yeast extract, casein hydrolysate, soybean meal alkali extract and so forth are used. In addition, phosphoric acid salts, carbonic acid salts, sulfuric acid salts, salts of magnesium, calcium, potassium, iron, manganese, zinc and so forth, specific amino acids, specific vitamins and so forth are used as required.

The culture temperature can be suitably changed within the range in which *Escherichia* bacteria grow and produce the GDH complex, and is preferably about 20 to 42°C. Although the culture time slightly varies depending on the conditions, it is sufficient that culture is terminated at an appropriate time by estimating the time when the maximum GDH complex yield is attained, and the culture time is usually about 12 to 72 hours. pH of the medium can be suitably changed within the range in which bacterial cells grow and produce the GDH complex, and is preferably within the range of about pH 6.0 to 9.0.

As the GDH complex, the culture broth as it is may be collected and used. However, when the GDH complex exists in the culture broth, it is generally used as a solution containing the GDH complex after separation from *Escherichia* bacterium cells by filtration, centrifugation or the like in a conventional manner. When the GDH complex exists in cells, it is isolated and collected as an aqueous solution by collecting the cells from the obtained culture by means of filtration, centrifugation or the like, then disrupting the cells by a physical method or an enzymatic method using lysozyme or the like, and adding a chelating agent such as EDTA and a surfactant, if necessary, to solubilize the subunits.

The GDH complex can be precipitated from the solution obtained as described above by, for example, vacuum concentration, membrane concentration, salting out using ammonium sulfate, sodium sulfate or the like, or fractional precipitation using a hydrophilic organic solvent such as methanol, ethanol or acetone. Furthermore, heat treatment and isoelectric precipitation are also effective purification means. Then, purification can be performed by a suitable combination of gel filtration using an adsorbent, gel filtration agent or the like, absorption chromatography, ion exchange chromatography, affinity chromatography and so forth to obtain a purified GDH complex. A purified enzyme preparation can be obtained by isolation and purification based on column chromatography.

The α-subunit of GDH solely exhibits the enzymatic activity. Therefore, the α-subunit alone can be isolated and purified from the *Escherichia* bacterium or the GDH complex of the present invention and used.

### Examples

The present invention will be explained more specifically with reference to the following examples.

### Example 1: Isolation of gene encoding GDH β-subunit of Burkholderia cepacia KS1 strain

### <1> Search of GDH β-subunit of Burkholderia cepacia KS1 strain

The GDH β-subunit gene derived from the KS1 strain was searched by using the *Burkholderia cepacia* J2315 strain genome database of the Sanger Centre (http://www.sanger.ac.uk/). With reference to the already elucidated N-terminus sequence of the GDH β-subunit of the KS1 strain (SEQ ID NO: 5), an amino acid sequence (SEQ ID NO: 6) having a homology to the cytochrome c subunits of alcohol dehydrogenases derived from *Acetobacter* sp. and *Gluconobacter* sp. (Tamaki T. et al., Biochem. Biophys. Acta, 1088(2): 292-300 (1991); Matsushita K. et al., Biosci. Biotech. Biochem., 56, 304-310 (1992); Takemura H. et al., J. Bacteriol., 175, 6857-66 (1993); Kondo K. et al., Appl. Environ. Microbiol., 63, 1131-8 (1997)), gluconate dehydrogenases derived from *Erwinia* sp. and *Pseudomonas* sp. (Yum D.Y. et al., J. Bacteriol., 179, 6566-72, (1997); Matsushita K. et al., J. Biochem., 85, 1173-81 (1979)), sorbitol dehydrogenase derived from *Gluconobacter* sp. (Choi E.S. et al., FEMS Microbiol. Lett., 125, 45-50 (1995)) and 2-ketogluconate dehydrogenases derived from *Erwinia* sp. and *Pantoea* sp. (Pujol C.J. et al., J. Bacteriol., 182, 2230-7 (2000)) was designed.

Using the aforementioned amino acid sequence as an index, gene sequences encoding amino acid sequences showing a high homology were searched from the aforementioned database of *Burkholderia cepacia* J2315 strain using BLAST. Then, five of the obtained sequences were examined for homology to the C-terminus sequence of the GDH α-subunit of the KS1 strain. As a result, amino acid sequences translated from two of gene fragments showed a high homology (> 90%). Because each gene fragment was as short as 200 to 500 bp, sequences showing a high homology with these sequences were searched from the genome database of the *Burkholderia cepacia* J2315 strain by using BLAST, and the fragments were joined. As a result, a fragment of 3110 bp was obtained. In the obtained nucleotide sequence, an ORF that appeared to be the one for the C-terminus of GDH and an ORF of 1275 bp that appeared to be the structural gene of cytochrome c existed. The obtained nucleotide sequence of the J2315 strain and the already cloned nucleotide sequence of the KS1 strain α-subunit were compared. The result showed that a nucleotide sequence showing a high homology to the nucleotide sequence encoding the signal peptide of J2315 strain cytochrome c was contained downstream from the α-subunit.

From the above, the third ORF (the nucleotide numbers 2386 and the followings in SEQ ID NO: 1) in the already cloned GDH gene of the *Burkholderia cepacia* KS1 strain (SEQ ID NO: 1, refer to WO 02/36779) was estimated to encode the β-subunit. Furthermore, because the amino acid sequence at the N-terminus of the purified β-subunit matched 5 amino acid residues translated from the nucleotide sequence of the nucleotide numbers 2452 to 2466 in SEQ ID NO: 1, the aforementioned ORF was considered to encode the β-subunit.

### <2> Amplification of β-subunit structural gene by inverse PCR

### (1) Culture of cells and extraction of genome

The KS1 strain was cultured overnight at 37°C in 5 ml of a complete medium (0.5% polypepton, 0.3% yeast extract, 0.5% NaCl) with shaking. The genome was extracted from the obtained cells using GennomicPrep^{™} Cells and Tissue DNA Isolation Kit (Amersham Pharmacia Biotech). The procedure was performed according to the attached manual. The obtained genome was subjected to a phenol/chloroform treatment and ethanol precipitation, and then dissolved in purified water.

### (2) Cyclization of genome fragments

The genome extracted from the KS1 strain was digested with *BamHI, EcoRI, HindIII, SmaI, SacI* and *XhoI*, and the genome fragments were collected by ethanol precipitation. A ligation reaction was performed overnight at 16°C for 1 µg of the genome digested with the restriction enzymes by using DNA Ligation Kit (Takara Shuzo).

### (3) PCR

In an amount of 50 pmol of a forward primer (EF1, SEQ ID NO: 7) and 50 pmol of a reverse primer (ER1, SEQ ID NO: 8) designed on the basis of the nucleotide sequence of the N-terminus signal sequence region of the GDH β-subunit of the KS1 strain (both the primers were synthesized by Invitrogen on commission), 0.5 µl of LATaq (Takara Bio Inc.), 8 µl of dNTP solution and 5 µl of 10 x PCR buffer were added with purified water to make the total volume of 50 µl, and PCR was performed using Program Temp Control System PC-801 (ASTEC). PCR was performed under the following condition. A cycle consisting of reactions at 94°C for 5 minutes, at 98°C for 20 seconds and at 62°C for 30 seconds was repeated 30 times, and followed by reactions at 72°C for 6 minutes and at 72°C for 10 minutes.

When the genome digested with the restriction enzyme *SmaI* was used as a template, a fragment of about 2.1 kbp was confirmed by agarose electrophoresis.

### <3> Sequencing of PCR-amplified fragment

### (1) TA cloning

The aforementioned inverse PCR product was subjected to agarose gel electrophoresis, and a band was excised and purified by using Gene Clean II Kit (Bio101 Inc.). This fragment was ligated to the pGEM-T vector by using pGEMR-T and pGEMR-T EASY Vector Systems (Promega). *Escherichia coli* DH5a was transformed with the ligated vector and cultured overnight in an L agar medium containing 50 µg/ml of ampicillin, 40 µg/ml of X-Gal and 0.1 µM IPTG. A white colony was selected among the colonies that emerged and cultured overnight in an L medium containing 50 µg/ml of ampicillin, and plasmids were extracted from the cells by the alkali method.

### (2) Preparation of sequence sample

The obtained plasmids were subjected to an RNase treatment, added with 20% PEG6000/2.5 M NaCl in a volume of 0.6 times the plasmids and left on ice for 1 hour. Then, the mixture was centrifuged at 15000 rpm at 4°C for 15 minutes to obtain a pellet. The pellet was washed with 70% ethanol, vacuum dried, and dissolved in purified water.

### (3) Analysis of DNA nucleotide sequence

The nucleotide sequence of the fragment inserted into the plasmid obtained in (2) was analyzed by using ABI PRISM^{™} 310 Genetic Analyzer (PERKIN-ELMER Applied Biosystems). A sequence of a part of the inserted fragment from the multi-cloning site of the vector was determined by using the M13 primer. As a result, a nucleotide sequence including the already elucidated sequence for the N-terminus of the β-subunit was confirmed. Primers were successively prepared on the basis of this sequence and used to determine the nucleotide sequence of the inserted fragment. The result is shown in SEQ ID NO: 9. Furthermore, the amino acid sequence encoded by the ORF included in this nucleotide sequence is shown in SEQ ID NO: 10.

The β-subunit consists of 425 amino acid residues in total. Among these, 22 residues are considered to be a signal peptide on the basis of comparison with the already obtained N-terminus amino acid sequence. The molecular weight calculated from the amino acid sequence was 45,276 Da, and the molecular weight excluding the signal peptide, 42,731 Da, was equivalent to the molecular weight of GDH β-subunit of the KS1 strain obtained by SDS-PAGE, 43 kDa. The heme binding motif (SEQ ID NO: 11) was confirmed at 3 sites in cytochrome c in the amino acid sequence of the β-subunit. This ORF located immediately downstream from the ORF of the α-subunit structural gene, and a sequence that appeared to be the SD sequence existed upstream from the start codon.

A homology search was performed for this amino acid sequence by using BLAST, and an overall high homology was observed at the amino acid level, i.e., a homology of 65% to the cytochrome c subunit of oxide reductase dehydrogenase derived from *Ralstonia solanacearum*, 48% to the cytochrome c subunit of sorbitol dehydrogenase derived from *Gluconobacter oxydans*, 44% to the cytochrome c subunit of gluconate dehydrogenase derived from *Eriwinia cypripedii* and 46.4% to the cytochrome c subunit of 2-ketogluconate dehydrogenase derived from *Pantoea citrea*. Furthermore, the heme binding motif sequence (SEQ ID NO: 11) was conserved in these amino acid sequences of cytochrome c.

The GDH β-subunit structural gene of the KS1 strain has homologies of 92.0% and 92.2% to the GDH β-subunit structural gene of the J2315 strain at the nucleotide sequence level and the amino acid level, respectively.

### Example 2: Introduction of GDHαβ gene into Escherichia coli and enhancement of ccm system

### <1> Preparation of chromosomal DNA from Burkholderia cepacia KS1 strain

Chromosomal genes were prepared from the *Burkholderia cepacia* KS1 strain in a conventional manner. That is, the bacterial strain was cultured overnight in a TL liquid medium (10 g of polypeptone, 1 g of yeast extract, 5 g of NaCl, 2 g of KH₂PO₄, 5 g of glucose in 1 L, pH 7.2) at 34°C with shaking. The proliferated cells were collected by centrifugation. The cells were suspended and treated at 50°C for 6 hours in a solution containing 10 mM NaCl, 20 mM Tris-HCl (pH 8.0), 1 mM EDTA, 0.5% SDS and 100 µg/ml of proteinase K. This suspension was added with an equivalent volume of phenol/chloroform, stirred at room temperature for 10 minutes and centrifuged to obtain a supernatant. This supernatant was added with sodium acetate to a final concentration of 0.3 M and overlaid with ethanol in a volume twice the volume of the supernatant to precipitate chromosomal DNA in the intermediate layer. It was scooped with a glass rod, washed with 70% ethanol and dissolved in a suitable volume of TE buffer to obtain a chromosomal DNA solution.

### <2> Preparation of GDHαβ gene

DNA fragments encoding the γ-subunit, the α-subunit and the β-subunit of GDH were amplified by PCR using the aforementioned chromosomal DNA as a template and oligonucleotides having the following sequences as primers.
<Forward primer: cF1>
5'-CATGCCATGGCACACAACGACAACAC-3' (SEQ ID NO: 12)
<Reverse primer: GDHbU1>
5'-GTCGACGATCTTCTTCCAGCCGAACATCAC-3' (SEQ ID NO: 13)

The C-terminus side of the amplified fragment was blunt-ended, then the N-terminus side was digested with *NcoI*, and the fragment was ligated to the similarly treated pTrc99A (Pharmacia). *E. coli* DH5α was transformed with the obtained recombinant vector, and a colony that emerged on an LB agar medium containing 50 µg/mL of ampicillin was collected. The obtained transformant was cultured in a liquid LB medium to extract the plasmids. When the inserted DNA fragment was analyzed, an inserted fragment of about 3.8 kb was confirmed. This plasmid was designated pTrc99Aγαβ. The GDHαβ gene in this plasmid is regulated by the trc promoter. pTrc99Aγαβ contains the ampicillin resistance gene.

### <3> Preparation of ccm system plasmid

pTrc99Aγαβ was digested with *EcoT22I*, and then the end was blunt-ended. Then, the fragment was digested with *NotI* and subjected to agarose gel electrophoresis to isolate and collect a short DNA fragment. This DNA fragment was inserted into pBBR122 digested with *ScaI* and *NotI* (MoBiTec) to prepare pBBGDHγαβ.

Chromosomal genes were prepared from *E. coli* JM109 in a conventional manner. That is, this bacterial strain was cultured overnight at 37°C in an LB medium (10 g of polypeptone, 5 g of yeast extract, 10 g of NaCl in 1 L, pH 7.0) with shaking. The proliferated cells were collected by centrifugation. The cells were suspended and treated at 50°C for 6 hours in a solution containing 10 mM NaCl, 20 mM Tris-HCl (pH 8.0), 1 mM EDTA, 0.5% SDS and 100 µg/ml of proteinase K. This suspension was added with an equivalent volume of phenol/chloroform, stirred at room temperature for 10 minutes and centrifuged to obtain a supernatant. This supernatant was added with sodium acetate to a final concentration of 0.3 M and overlaid with ethanol in a volume twice the volume of the supernatant to precipitate chromosomal DNA in the intermediate layer. It was scooped with a glass rod, washed with 70% ethanol and dissolved in a suitable volume of TE buffer to obtain a JM109 chromosomal DNA solution.

The DNA fragment encoding the ccm system gene was amplified by PCR using the JM109 chromosomal DNA as a template and oligonucleotides having the following sequences as primers.
<Forward primer: ECccmDl>
5'-TGGCCATGGTTGAAGCCAGAGAGTTACTTT-3' (SEQ ID NO: 14)
<Reverse primer: ECccmU1>
5'-TTATTTACTCTCCTGCGGCGACAAATGTTG-3' (SEQ ID NO: 15)

The amplified end was blunt-ended, and then the N-terminus side was digested with *NcoI*. This fragment was digested with *ACCI*, blunt-ended, and then ligated to pBBGDHγαβ digested with NcoI to prepare pBBJMccm. The GDHαβ gene was removed by the digestion with *AccI*, blunt-ending and the subsequent digestion with *NcoI* of pBBGDHγαβ.

### <4> Introduction of GDHαβ gene and ccm system into Escherichia coli

*E. coli* JM109 was transformed with pTrc99Aγαβ and pBBJMccm to obtain JM109/pTrc99Aγαβ,pBBJMccm. Furthermore, as a control, *E. coli* JM109 was transformed with pTrc99Aγαβ to obtain JM109/pTrc99Aγαβ.

These transformants were cultured overnight in 10 mL of 2 x YT medium containing 50 µg/ml of ampicillin and 50 µg/ml of kanamycin (JM109/pTrc99Aγαβ,pBBJMccm), or 50 µg/ml of ampicillin (JM109/pTrc99Aγαβ) at 34°C with shaking. Furthermore, the *Burkholderia cepacia* KS1 strain was cultured overnight in 10 mL of a complete medium with shaking. The cells were collected from a part of each culture broth by centrifugation, added with 10 mM potassium phosphate buffer (pH 7.0) containing 1% sodium cholate to the volume before the centrifugation and then disrupted by ultrasonication. Then, the GDH activity in the supernatant obtained by centrifuging the disrupted suspension was determined.

The GDH activity was determined by the following procedure. A mixture of 47 mM phosphate buffer (pH 6.0), 20 mM glucose, 2 mM phenazine methosulfate and 0.1 mM 2,6-dichlorophenol indophenol was heated at 37°C beforehand and added to a sample to start a reaction. While the mixture was maintained at 37°C, changes in absorbance at 600 nm were measured to determine the activity. The GDH activity was obtained by using a molecular extinction coefficient (4.76 mM/cm) of 2,6-dichlorophenol indophenol. One unit (U) of the enzymatic activity was defined as the oxidization amount of 1 µmol of 2,6-dichlorophenol indophenol per minute.

As a result, GDH activities of JM109/pTrc99Aγαβ, *Burkholderia cepacia* KS1 and JM109/pTrc99Aγαβ,pBBJMccm were 0.3, 1.4 and 32 U/mL, respectively. That is, JM109/pTrc99Aγαβ hardly exhibited the GDH activity, and *Burkholderia cepacia* KS1, a wild strain, exhibited a weak GDH activity. However, JM109/pTrc99Aγαβ,pBBJMccm exhibited an extremely high GDH activity. In addition, the GDH complex was purified from JM109/pTrc99Aγαβ,pBBJMccm and subjected to SDS-PAGE. As a result, it could be confirmed that the GDH complex showed the same electrophoresis pattern as that of the GDH complex purified from the KS1 strain (Fig. 1).

### Industrial Applicability

According to the present invention, an enzyme complex containing the α-subunit and the β-subunit of glucose dehydrogenase of *Burkholderia cepacia* can be abundantly expressed in an *Escherichia* bacterium.

## Claims

1. An *Escherichia* bacterium, into which is introduced DNA encoding the α-subunit and the β-subunit of glucose dehydrogenase (GDH) of *Burkholderia cepacia* in an expressible form and a plasmid containing the cytochrome c maturation (ccm) operon of *Escherichia coli* ligated to a suitable promoter which constitutively expresses the operon, wherein expression of the ccm system is enhanced, as compared with a wild-type strain or unmodified strain of *Escherichia* bacteria.

2. The *Escherichia* bacterium according to claim 1, wherein the DNA encoding the α-subunit locates upstream from the DNA encoding the β-subunit, and expressions of them are regulated by a single promoter.

3. The *Escherichia* bacterium according to claim 1, which is further introduced with a DNA encoding the γ-subunit of the glucose dehydrogenase in an expressible form.

4. The *Escherichia* bacterium according to claim 3, wherein the DNA encoding the γ-subunit locates upstream from the DNA encoding the α-subunit.

5. The *Escherichia* bacterium according to any one of claims 1 to 4, wherein the *Escherichia* bacterium is *Escherichia coli.*

6. A method for producing a glucose dehydrogenase complex, which comprises culturing the *Escherichia* bacterium according to any one of claims 1 to 5 so that the DNAs encoding the α-subunit and the β-subunit are expressed and the glucose dehydrogenase complex is produced, and collecting the complex.

## Patentansprüche

1. *Escherichia*-Bakterium, in das DNA, in exprimierbarer Form, die für die α-Untereinheit und die β-Untereinheit von Glucosedehydrogenase (GDH) von *Burkholderia cepacia* kodiert, und ein Plasmid, das das Operon für die Cytochrom c-Reifung (ccm) von *Escherichia coli* ligiert an einen geeigneten Promoter, der das Operon konstitutiv exprimiert, enthält, eingeschleust ist, wobei die Expression des ccm-Systems im Vergleich mit einem Wildtyp-Stamm oder einem unmodifizierten Stamm von *Escherichia*-Bakterien erhöht ist.

2. *Escherichia*-Bakterium nach Anspruch 1, wobei die DNA, die für die α-Untereinheit kodiert, stromaufwärts von der für die β-Untereinheit kodierenden DNA liegt und die Expression von diesen durch einen einzigen Promotor reguliert wird.

3. *Escherichia*-Bakterium nach Anspruch 1, in das ferner DNA, in exprimierbarer Form, eingeschleust ist, die für die γ-Untereinheit der Glucosedehydrogenase kodiert.

4. *Escherichia*-Bakterium nach Anspruch 3, wobei die für die γ-Untereinheit kodierende DNA stromaufwärts von der für die α-Untereinheit kodierenden DNA liegt.

5. *Escherichia*-Bakterium nach einem der Ansprüche 1 bis 4, wobei das *Escherichia*-Bakterium *Escherichia coli* ist.

6. Verfahren zur Herstellung eines Glucosedehydrogenase-Komplexes, welches umfasst: Kultivieren des *Escherichia*-Bakteriums nach einem der Ansprüche 1 bis 5, so dass die für die α-Untereinheit und die β-Untereinheit kodierenden DNAs exprimiert werden und der Glucosedehydrogenase-Komplex gebildet wird, und Gewinnung des Komplexes.

## Revendications

1. Bactérie *Escherichia* dans laquelle sont introduits un ADN codant pour la sous-unité α et la sous-unité β de la glucose déshydrogénase (GDH) de *Burkholderia cepacia* sous une forme exprimable et un plasmide contenant l'opéron de maturation du cytochrome C (ccm) d'*Escherichia coli* ligaturé à un promoteur approprié qui exprime l'opéron de façon constitutive, dans laquelle l'expression du système de ccm est amplifiée par comparaison à une souche de type sauvage ou une souche non modifiée d'une bactérie *Escherichia.*

2. Bactérie *Escherichia* selon la revendication 1, dans laquelle l'ADN codant pour la sous-unité α est localisé en amont de l'ADN codant pour la sous-unité β, et leurs expressions sont régulées par un seul promoteur.

3. Bactérie *Escherichia* selon la revendication 1, dans laquelle est introduit en outre un ADN codant pour la sous-unité γ de la glucose déshydrogénase sous une forme exprimable.

4. Bactérie *Escherichia* selon la revendication 3, dans laquelle l'ADN codant pour la sous-unité γ est localisé en amont de l'ADN codant pour la sous-unité α.

5. Bactérie *Escherichia* selon l'une quelconque des revendications 1 à 4, la bactérie *Escherichia* étant *Escherichia coli.*

6. Procédé de production d'un complexe de glucose déshydrogénase, comprenant la culture de la bactérie *Escherichia* selon l'une quelconque des revendications 1 à 5 de façon à ce que les ADN codant pour la sous-unité α et la sous-unité β soient exprimés, et la récupération du complexe.
